# EUROPÄISCHE PATENTSCHRIFT

(11) **EP 4 317 389 B1**
(45) Veröffentlichungstag und Bekanntmachung des Hinweises auf die Patenterteilung: **27.05.2026**
(21) Anmeldenummer: 23169961.2
(22) Anmeldetag: 26.04.2023
(51) Int. Cl.: C11D 7/32, B01D 53/14, B01D 53/44, B01D 53/77, C11D 3/00, C11D 7/26, A61K 8/27, A61K 8/44, A61L 9/01

(54) **LUFTSCHADSTOFFNEUTRALISATIONSREINIGER (AIR CLEANER) MIT GERUCHSABSORBIERENDER FUNKTION ZUR VERBESSERUNG DER RAUMLUFT**
AIR POLLUTANT NEUTRALIZERS WITH ODOR ABSORBING FEATURE TO IMPROVE ROOM AIR
PURIFICATEUR DE NEUTRALISATION DES POLLUANTS DE L'AIR (AIR-CLEAN) DOTÉ D'UNE FONCTION D'ABSORPTION DES ODEURS POUR AMÉLIORER L'AIR AMBIANT

(30) Priorität: 04.08.2022 DE 202022104440 U
(43) Veröffentlichungstag der Anmeldung: 07.02.2024
(73) Patentinhaber: Neutec Chemie GmbH, 55294 Bodenheim (DE); Linden Chemie GmbH & Co.KG, 53925 Kall (DE)
(72) Erfinder: Wedell, Ronni, 55294 Bodenheim (DE); Vossen, Jürgen, 53925 Kall (DE)
(74) Vertreter: Mackert, Andreas

(56) Entgegenhaltungen:
- CN-A- 107 418 431
- CN-A- 108 159 865
- CN-A- 111 298 639
- US-A1- 2015 118 173
- ANONYMOUS: "Polyfix ZRC 25 GP - Concentrate of a Broad Spectrum Odour Absorber; Technical Data Sheet", SCHILL + SEILACHER, 29 June 2018 (2018-06-29), XP093109644, Retrieved from the Internet <URL:https://rossorg.com/wp-content/uploads/2019/09/Polyfix-ZRC-25-GP.-TDS.-06.29.18.pdf> [retrieved on 20231206]

## Beschreibung

Die Erfindung betrifft einen Luftschadstoffneutralisationsreiniger (Air Cleaner), der Schadstoffe absorbiert und Gerüche neutralisiert und so zur Verbesserung der Raumluft beiträgt.

Innenraumbelastungen mit Schadstoffen sind in zunehmendem Maße für Befindlichkeitsstörungen von Menschen verantwortlich, wobei chronische Belastungen und Kombinationswirkungen von Schadstoffen im Niedrigdosisbereich hierbei im Vordergrund stehen.

Insbesondere Formaldehydbelastungen und Aldehyde durch Zigarettenrauch, Farben, Lacke, Kleber, Desinfektionsmittel, heiße Fette uvm. belasten die Raumluft und somit häufig auch die Gesundheit der in diesen Räumen lebenden oder arbeitenden Menschen. Hinzu kommt, daß auch Möbel und sonstige Einrichtungsgegenstände sowie Bodenbeläge Schadstoffe kontinuierlich an die umgebende Raumluft abgeben können.

Zu den schwerflüchtigen organischen Verbindungen gehören neben Pestiziden und Holzschutzmitteln, die in Innenräumen überwiegend aus Quellen im Raum selbst stammen, Dioxine, Furane, polycyclische aromatische Kohlenwasserstoffe (PAK, PAH) und deren Derivate, die in Innenräumen und in der Außenluft vorkommen.

Isocyanate sind organische Verbindungen die mit Phenolen und Alkoholen zu Polyurethanen reagieren. Sie finden sich in Dispersionen, Zweikomponenten- und anderen Klebern auf Lacken und Schaumstoffen Verwendung. Die Reaktion läuft jedoch nicht vollständig ab, so dass Restmonomere im fertigen Produkt enthalten sind, die an die Innenraumluft abgegeben werden.

Vor diesem Hintergrund werden Anstrengungen unternommen, die Raumluft von dieser Vielzahl von Belastungen zu reinigen und somit gesundheitlichen Problemen der Menschen vorzubeugen, die sich in den industrialisierten Ländern Europas etwa 80% bis 90% des Tages in Innenräumen aufhalten und somit den Schadstoffen ausgesetzt sind. Gleichzeitig wird bei Einsatz von Duftstoffen auf Basis von ausschließlich natürlichen oder ätherischen Ölen die Raumluft mit angenehmem Geruch auf natürlich Weise angereichert.

Die DE 102 12 983 C1 offenbart beispielsweise einen Sanierungsbaustoff für schadstoffbelastete Gebäude bestehend aus einer Trockenmasse aus Tonmehl und Sand, dem Schafwollfasern zugesetzt sind, deren reaktive Fläche durch Schneiden kurzer Faserlängen und Aufbrechen einer oder mehrerer Faserschichten, insbesondere der Keratinschicht, vergrößert worden ist, wobei die Schafswollfasern Schadstoffe aus der Raumluft binden sollen.

Aus der Veröffentlichung US 5 705 537 A ist als anderer Baustoff ein Phenolharzschaum bekannt, der als Isolierung verwendet werden kann. Es handelt sich hier um ein geschäumtes Phenol-Formaldehyd-Resol-Harz, das ein Peptid, ein proteinhaltiges Material, Cystein, Glutaminsäure, Glycin, Isoleucin, Leucin, Lysin, Phenylalanin, Serin, Tryptophan, oder Mischungen davon in einer Menge enthält, die wirksam eine Reduzierung von Emissionen von freiem Formaldehyd aus dem geschäumten Harz bewirken.

In der offenbarten Erfindung der CA 1 162 904 A wird ein Verfahren der Chemiesorption eingesetzt, um gasförmiges Formaldehyd zu festigen, in nicht flüchtigen Verbindungen umzuwandeln und in den Absorber zu binden. Der Absorber kann hierfür in verschiedenen Formen von Filtern hergestellt werden, und eignet sich für beruflichen Atemschutz, Luft-Reinigung in Arbeits- und Wohnbereichen und für die Formaldehyd-Absorption von Prozessgasen. Der Absorber kann hier auch präventiv in Kunstharzen zur Reduzierung von Formaldehyd-Emissionen integriert werden.

Aus der Veröffentlichung CN 106 589 475 A schließlich ist ein wasserlösliches Shitosan-Kombinationsmittel bekannt, mit dem die Verwendungskosten von Shitosan gesenkt und die Verwendungswirkung von Shitosan erhöht werden sollen, wofür ein hydrotropes Shitosan-Material vorgeschlagen wird, das schnell wasserlöslich ist und Eigenschaften wie relativ niedrige Kosten, Ungiftigkeit und Umweltverträglichkeit aufweist. Das so gewonnene Shitosan-Kombinationsmittels kann in die Luft gesprüht oder einem Luftbefeuchter zugegeben werden, um Formaldehyd aus der Luft zu entfernen, Luftdunst zu beseitigen und Bakterienviren in der Luft zu unterdrücken. CN 107 418 431 A und CN 108 159 865 A offenbaren eine wässrige Formulierung, die Aminosäuren und Zinkricinoleat enthält, um üble Gerüche zu bekämpfen.

Dies berücksichtigend ist es die Aufgabe der vorliegenden Erfindung, einen verbesserten Luftschadstoffneutralisationsreiniger (Air Cleaner) zu schaffen, der eine konstante Neutralisierung von Gerüchen in der Raumluft ermöglicht und hierbei Schadstoffe wie Formaldehyde und Aldehyde und andere Schadstoffe katalysiert. Der Luftschadstoffneutralisationsreiniger soll hierbei sehr absorptionsfähig und langfristig wirksam, gleichzeitig aber auch einfach und ungefährlich in der Anwendung sein. Zudem sollen möglichst hohe Standards in Bezug auf natürliche Inhaltsstoffe und somit die ökologische Verträglichkeit des Produktes verwirklicht werden, die eine Zertifizierung nach ECOCERT-Standard ermöglichen.

Die Aufgabe wird gelöst durch den erfindungsgemäßen Luftschadstoffneutralisationsreiniger (Air Cleaner) mit den Merkmalen des Anspruchs 1.

Die Unteransprüche haben vorteilhafte Weiterbildungen des Luftschadstoffneutralisationsreinigers (Air Cleaners) zum Gegenstand.

Der erfindungsgemäße Luftschadstoffneutralisationsreiniger (Air Cleaner) verwirklicht die Aufgabenstellung zum einen durch einen neuartigen Aminosäurekomplex, wodurch mit erhöhter Wirksamkeit Schadstoffe absorbiert und Gerüche neutralisiert werden und so eine Verbesserung der Raumluft erfolgt. Ein Zinkrizinoleat Compound bewirkt hierbei insbesondere die angestrebte Neutralisierung von Geruchsstoffen zur Verbesserung der Raumluft.

Es wurde durch experimentelle Versuchsreihen ein erfindungsgemäßer Aminosäurenkomplex zumindest umfassend Bio-L-Arginin, L-Lysin, L-Glutamin und L-Prolin und ein Compound auf Zinkrizinoleat-Basis von bis zu 10 % als besonders vorteilhaft festgestellt, um diese Aufgabenstellung als Bestandteil eines Luftreinigers zu verwirklichen. Dieser im erfindungsgemäßen Luftschadstoffneutralisationsreiniger enthaltene Aminosäurenkomplex in der offenbarten Wirkstoffkombination, weist aufgrund der Wechselwirkung bzw. dem Zusammenwirken der offenbarten Bestandteile gegenüber bekannten Verbindungen veränderte physikalisch-chemische Parameter und daraus resultierend veränderte biologische Eigenschaften bzw. Wirksamkeiten auf, wodurch sich gezeigt hat, dass dieser Aminosäurenkomplex die angestrebte Aufgabenstellung bei der Schadstoffneutralisation besser erfüllen können. Der erfindungsgemäße Luftschadstoffneutralisationsreiniger ist hierdurch in der Lage, mit den Luftschadstoffen chemisch zu reagieren und diese wirksam zu neutralisieren. Zudem werden unangenehme Geruchsstoffe in ihrer Wirkung vermindert bzw. durch enthaltene Duftstoffe in Form eines natürlichen und/oder ätherischen Öles überlagert.

Das Zinkrizinoleat Compound bewirkt zudem eine sofortige Absorption der meisten gängigen und für den Menschen wahrnehmbaren unangenehmen Geruchsmoleküle, wie Schwefelverbindungen (Thiole, Thioether, Mercaptane), Stickstoffverbindungen (Amine) und Carbonsäuren. Durch einen Chelatbildner wird das Zinkrizinoleat aktiviert, wird grenzflächenaktiv und umschließt und neutralisiert das Geruchsmolekül effektiv und direkt in der Gasphase.

Das heißt, ein wesentlicher Vorteil des erfindungsgemäßen Luftschadstoffneutralisationsreinigers ist, dass hier der Aminosäurenkomplex wirksam ist gegen Aldehyde in der Raumluft und das Zinkrizinoleat Compound unterstützend und ergänzend unmittelbar Gerüche neutralisiert. Auf diese Weise ist ein umfassendes Wirkspektrum in einem Luftschadstoffneutralisationsreiniger vereint, der effektiv Schadstoffe und Gerüche bekämpft.

Die erfindungsgemäße Zusammensetzung ist definiert durch Anteil-Unter- und Obergrenzen in der nachfolgenden Tabelle A dargestellt:

**Tabelle A**

| **Inhaltsstoff** | **Anteils-Untergrenze** | **Anteils-Obergrenze** |
|---|---|---|
| Wasser | 70,00% | 90,00% |
| Bio-Ethanol | 6,00% | 15, 00% |
| Bio-L-Arginin | 0,50% | 3,00% |
| L-Glutamin | 0,05% | 1,5% |
| L-Lysin | 0,30% | 2,00% |
| L-Prolin | 0, 04 | 1,00% |
| Polyglyceryl | 2,00% | 5,00% |
| Zitronensäure | 0,20% | 1,00% |
| Zinkrizinoleat Compound | 5,00% | 10,00% |
| | **Summe: 100,00%** | |

Die hohe Wirksamkeit wird in Bezug auf die Neutralisierung von Gerüchen beim Zinkrizinoleat Compound wie ausgeführt durch einen Chelatbildner bewirkt, der das Zinkrizinoleat aktiviert und grenzflächenaktiv macht. Durch eine vergrößerte Oberfläche werden die Geruchsmoleküle umschlossen. Als Chelatbildner wird der Inhaltsstoff Tetrasodium Glutamate Diacetate als biologischer Chelatbildner weitestgehend pflanzlichen Ursprungs eingesetzt, was die Zertifizierbarkeit nach ECOCERT Standard unterstützt.

Bei einer vorteilhaften Ausführungsform des erfindungsgemäßen Luftschadstoffneutralisationsreinigers hat sich hierbei ein Mengenanteil Tetrasodium Glutamate Diacetate von etwa 0,8% bis 2,0% an der Gesamtmenge der Mischung als funktionsfördernd herausgestellt. Auch L-Glutamin ist bei einer vorteilhaften Ausführungsform des erfindungsgemäßen Luftschadstoffneutralisationsreinigers als Bestandteil von 0,05% bis 1,5% in der Gesamtmenge der Mischung enthalten.

Bei der Formulierung des erfindungsgemäßen Luftschadstoffneutralisationsreinigers unter Berücksichtigung der Problemstellung, eine hohe Wirksamkeit in Bezug auf die Neutralisierung von Gerüchen und Schadstoffen in der Raumluft zu erreichen und gleichzeitig möglichst hohe Standards in Bezug auf natürliche Inhaltsstoffe und somit die ökologische Verträglichkeit des Produktes zu verwirklichen, ergaben sich zu überwindende Herausforderungen, eine phasenstabile und klargestellte Mischung zu erreichen.

Unter einer phasenstabilen oder phasenfreien Verbindung im Sinne der Erfindung ist zu verstehen, dass das Produkt homogen ist und es somit nur eine Phase gibt. Das bedeutet, dass es nur einen Aggregatzustand aufweist und als homogenes Gemisch einphasig vorliegt.

Dieser Zustand wurde experimentell durch den erfindungsgemäßen Luftschadstoffneutralisationsreiniger der angeführten Formulierung erreicht. Insbesondere ist hier der optionale Zusatz natürlicher Duftstoffe, insbesondere ätherischer Öle, problematisch. Aufgrund der Aufgabenstellung, möglichst hohe Standards in Bezug auf natürliche Inhaltsstoffe zu erfüllen, sind Duftstoffe beispielsweise in Form ätherischer Öle als Naturprodukte zu verwenden, was die Herausforderung vergrößert, ein homogenes und phasenstabiles Produkt zu schaffen. Die angegebenen Aminosäuren in deren angegebenen Anteilen an der Mischung und in Kombination mit polyglycerol als Emulgator und/oder zur Regelung der Viskosität hat sich als Kombination gezeigt, die diese angestrebte phasenstabile Homogenität der Mischung der natürlichen Inhaltsstoffe bei hoher Wirksamkeit in Bezug auf die Neutralisierung von Gerüchen und Schadstoffen realisiert.

Unter der klargestellten Mischung im Sinne der vorliegenden Erfindung ist zu verstehen, dass in der als Produkt vorliegenden Flüssigkeit ein optisch trüber Eindruck vermieden wird. Eine Trübung durch kleine Partikel in der Flüssigkeit, die eine vom Trägerstoff abweichende Brechzahl besitzen, soll so vermieden werden, da dies als Anwendererwartung für den vorliegenden Luftschadstoffneutralisationsreiniger besteht und ein trübes Produkt weniger Akzeptanz erfährt.

Auch für diese Anforderung an das Produkt hat die Auswahl der angegebenen Aminosäuren als Aminosäuren-Komplex in deren angegebenen Anteilen an der Mischung in Kombination mit Polyglyceryl gute Ergebnisse erzielt. Die Mischung konnte auch bei der angestrebten Verwendung der rein natürlichen Inhaltsstoffe klargestellt werden, ohne Einbußen in Bezug auf eine hohe Wirksamkeit in Bezug auf die Neutralisierung von Gerüchen und Schadstoffen in Kauf nehmen zu müssen.

Eine andere durch die Verwendung der natürlichen Inhaltsstoffe zu überwindende Problematik im Bereich des Aminosäuren-Komplexes ist der pH-Wert, der für die angestrebt hohe Wirksamkeit in Bezug auf die Neutralisierung von Gerüchen und Schadstoffen in der Raumluft tendenziell im neutralen Bereich liegen sollte. Das Arginin im erfindungsgemäßen Luftschadstoffneutralisationsreiniger wird in Form von Bio-L-Arginin verwendet. Im Stand der Technik wird Arginin in beispielsweise Reinigungslösungen als sogenanntes HCL-Produkt eingesetzt, wobei hier der Arginin-Gehalt deutlich geringer ist aber annähernd neutral verliegt und die Aminosäure im Salz der Salzsäure (HCL) gebunden ist.

Das erfindungsgemäß verwendete Bio-L-Arginin zählt zu den basischen Aminosäuren mit nahezu 100 % der Aminosäure und insofern einer deutlich höheren Konzentration und Reinheit der Aminosäure, was sich als positiv für die angestrebt hohe Wirksamkeit in Bezug auf die Neutralisierung von Gerüchen erwiesen hat. Die Bio-L-Arginin Aminosäure liegt erfindungsgemäß in Food-Grade-Qualität vor, was das Produkt zusätzlich in Bezug auf Gesundheits- und Umweltverträglichkeit hin verbessert.

Im Gegensatz zu dem im Stand der Technik verwendeten Arginin in HCL Qualität und mit einem in etwa neutralen PH-Wert weist Bio-L-Arginin einen basischen pH-Wert von etwa 11 auf, wobei es für die Wirksamkeit der Aminosäuren als Schadstoffbinder oder -neutralisierer zentral ist, dass das Produkt in etwa einen neutralen pH-Wert von 7 aufweist. Es hat sich hierbei als vorteilhaft herausgestellt, in die vorliegende Formulierung den Inhaltsstoff der Zitronensäure aufzunehmen, die als pH-Wert-Einsteller verwendet wird, um so den pH-Wert auf den neutralen Wert von etwa 7 einzustellen. Auch dies ist demnach ein wichtiger Aspekt der Formulierung, um ECOzertifizierte Inhaltsstoffe mit hoher Wirksamkeit in einem Produkt zusammenzuführen.

Die Mengenverhältnisse des Aminosäurenkomplexes mit den Verhältnissen der Aminosäuren zueinander wie auch das Verhältnis des polyglycerol als Emulgator und/oder zur Regelung der Viskosität zum Aminosäurenkomplex haben sich als besonders positiv erwiesen, um eine hohe Wirksamkeit mit einer optimalen Umweltverträglichkeit in Einklang zu bringen. Desweiteren hat sich das Mengenverhältnis der Zitronensäure in der Mischung gegenüber dem Aminosäurenkomplex als ideal gezeigt, um über den neutralen pH-Wert die Wirksamkeit zusätzlich zu verbessern.

Die in Tabelle A angegebenen Inhaltsstoffe sind hierbei nicht abschließend zu verstehen, da weitere Inhaltsstoffe zugesetzt werden können. Dies betrifft beispielsweise Duftstoffe in Form ätherischer Öle, auf die bereits eingegangen wurde. Bei den angegebenen anteiligen Mengenangaben wird dies je nach Duftstoffmengenanteil beispielsweise zu einer Reduzierung des Wasseranteils führen. Auch die weiteren Anteile der Inhaltsstoffe können sich hierdurch geringfügig verändern.

Der erfindungsgemäße Luftschadstoffneutralisationsreiniger (Air Cleaner) nach Anspruch 1 unterscheidet sich grundsätzlich sowohl im Einsatzprofil als auch im Wirkungsspektrum von herkömmlichen Luftreinigern. Es ist für den Abbau von Reiz- und Geruchsstoffen in der Innenraumluft durch Chemieabsorption optimiert und enthält synergistisch wirkende Komponenten eines Aminosäurenkomplexes, das als Absorptionsmittel insbesondere für gasförmige, proteinreaktive Substanzen in Innenräumen zum Einsatz kommt. Hierzu gehören u.a. Aldehyde und VOC (volatile organic compounds = flüchtige organische Verbindungen).

Der Luftschadstoffneutralisationsreiniger (Air Cleaner) dient so der Aufnahme und Abreaktion von Reiz- und Geruchstoffen belasteter Innenräume und der Prävention derartiger Belastungen.

Die wesentlichen Verbesserungen, die durch den erfindungsgemäßen Luftschadstoffneutralisationsreiniger erzielt werden, sind nachfolgend nicht abschließend und beispielhaft angeführt.
- Abbau von unter anderem Formaldehydverbindungen.
- Das Vermeiden externer Quellen wie Luftfilter, beispielsweise Schwebstofffilter wie HEPA-Filter (HEPA = High-Efficiency Particulate Air/Arrestance), Aktivkohlefilter oder UV-C-Luftreiniger- oder UV-Luftdesinfektionsgeräte, wie auch Licht, Sauerstoff oder Ionentauscher, mit denen die Luft gemäß zuvor beschriebener Anwendung gereinigt wird, wird erreicht.
- Eine Mehr-Stunden-Funktionalität bzw. Langzeitwirkung wird bei einer Kombination mit beispielsweise Lufterfrischern oder Luftwäschern ermöglicht.
- Der Abbau und die Neutralisation von insbesondere proteinreaktiven Reiz- und Geruchstoffen konnte nachgewiesen werden.
- Reaktions- und Reaktionszwischenprodukte wurden experimentell als unbedenklich identifiziert.
- Es werden 99,5 % natürliche Rohstoffe verwendet, deren biologische Abbaubarkeit nachgewiesen ist. Somit erfolgt eine Zertifizierung nach dem hohen ECOCERT Standard "Wasch- und Reinigungsmittel".
- Es erfolgt die Anwendung des neuartigen Aminosäurewirkstoffkomplexes, der in Kombination mit den weiteren Inhaltsstoffen zum einen die hohe Wirksamkeit und zum anderen den hohen ECOCERT Standard ermöglicht.

Der erfindungsgemäße Luftschadstoffneutralisationsreiniger (Air Cleaner) bewirkt somit nachweislich positive Auswirkungen in Bezug auf ein verbessertes Raumklima durch Abbau von Geruchs- und Schadstoffen bei gleichzeitig deutlich verbesserter Umweltverträglichkeit.

Technisch neu im Vergleich zu bestehenden Luftreinigungs-Lösungen, beispielsweise durch Aktivkohlefilter, ist zudem, dass Reiz-, Schad- und Geruchsstoffe chemisch umgesetzt, dauerhaft gebunden und im Fall von proteinreaktiven Verbindungen neutralisiert werden unter Vermeidung von Geruchs- bzw. Schadstoff Überdeckungen.

Der Einsatz eines mit ECOCERT zertifizierten Rohstoffen und einem derartig zusammengesetzen Aminosäurenkomplexes in einem Luftreiniger ist bisher im Stand der Technik nicht offenbart oder nahegelegt. Zudem unterliegt die Zertifizierung unter dem ECOCERT "Wasch- und Reinigungsmittel" Standard höchsten Ansprüchen an die 99,5 % natürlichen Inhaltsstoffe und ebenso an die Nachhaltigkeit des gesamten Produktes. Dieser Standard stellt höchste Ansprüche an die natürliche Herkunft aller Rohstoffe des Produktes und einen nachgewiesenen biotechnologischen Herstellungsprozess jedes einzelnen Rohstoffes. Gentechnische Veränderungen sind in diesem Standard vollumfänglich ausgeschlossen.

Zudem kann das Produkt mit verschiedenen Varianten von ECOCERT zertifizierten natürlichen ätherischen Duftstoffen angereichert werden, um die Funktionsumfang des Gesamtproduktes um die Raumbeduftung zu erweitern. Die Wirkung der Duftstoffe wird durch das Zinkrizinoleat hierbei vorteilhafterweise nicht beeinträchtigt.

Die Erfindung umfasst somit folgende Funktionsweisen, welcher unter dem ECOCERT Standard "Wasch- und Reinigungsmittel" zertifiziert und getestet wurden:
- Absorption und dauerhafte Reduzierung von Luftschadstoffen;
- Sofortige Absorption von Gerüchen;
- Raumbeduftung.

Der Einsatzbereich des Produktes umfasst nachfolgende Einsatzmöglichkeiten:
- Einsatz in einem Raumspray;
- Einsatz als Konzentrat im Wasseranteil eines Luftreinigungsgerätes;
- Einsatz als Formulierungszusatz bei anderen Reinigern, z.B. für Böden, Oberflächen, Waschmaschinen o.ä. nach Detergenzienverordnung.

Die folgende tabellarische Aufstellung betrifft Geruchsstoffe und deren Eigenschaften, die durch das erfindungsgemäß in Luftschadstoffneutralisationsreiniger eingesetzte Compound auf Zinkrizinoleat-Basis mit einer weiteren Aminosäure neutralisiert werden können. Diese Liste ist hierbei lediglich zur Verdeutlichung beispielhaft zu verstehen und ist weder vollständig noch abschließend zu bewerten.

**Tabelle B von neutralisierbaren Geruchsstoffen:**

| | **Formel** | **Charakteristischer Geruch** | **Geruchsschwelle (ppm)** | **Wahrnehmung (ppm)** | **Molekulargewicht** |
|---|---|---|---|---|---|
| Allylmerchaptan | CH₂·CH·CH₂·SH | Kaffee stark nach Knoblauch, | 0,0005 | --- | 74,15 |
| Ammoniak | NH₃ | stechend scharf | 0,037 | 46,8 | 17,03 |
| Amylmerchaptan | CH₃·(CH₂)₃·CH₂·SH | Faulig | 0,0003 | --- | 104,22 |
| Benzylmerchaptan | C₆H₅·CH₂·SH | stark unangenehm | 0,00019 | --- | 124,21 |
| Butylamin | C₂H₅·CH₂·CH₂·NH₂ | sauer, ammoniakartig | --- | 0,24 | 73,14 |
| Cadaverin | H₂N·(CH₂)₅·NH₂ | Faul nach Verwesung | --- | --- | 102,18 |
| Merchaptan Crotyl | CH₃·CH:CH·CH₂·S | nach Stinktier | 0,000029 | --- | 90,19 |
| Dibutylamin | (C₄H₉)₂NH | fischig | 0,016 | --- | 129,25 |
| Diisopropylamine | (C₃H₇)₂NH | fischig | 0,0035 | 0,085 | 101,19 |
| Dimethylamin | (CH₃)₂NH | verfault, fischig | 0,047 | 0,047 | 45,08 |
| Dimethylsulfid | (CH₃)₂S | faules Gemüse | 0,001 | 0,001 | 62,13 |
| Diphenylsulfid | (C₆H₅)₂S | Sehr unangenehm | 0,000048 | 0,0021 | 186,28 |
| Äthylamin | C₂H₅·NH₂ | Ammoniakartig | 0,83 | 0,83 | 45,08 |
| Ethylmerchaptan | C₂H₅·SH | Fauler Kohl | 0,00019 | 0,001 | 62,1 |
| Wasserstoffsulfid | H₂S | faule Eier | 0,00047 | 0,0047 | 34,1 |
| Indol | C₂H₆NH₂ | Sehr unangenehm | --- | --- | 117,15 |
| Methylamin | CH₃NH₂ | Faul & fischig | 0,021 | 0,021 | 31,05 |
| Methyl- Merchaptan | CH₃SH | Fauler Kohl | 0,0011 | 0,0021 | 48,1 |
| Propyl- Merchaptan | CH₃·CH₂·CH₂·SH | Unangenehm | 0,000075 | --- | 76,16 |
| Putrescin | NH₂(CH₂)₄NH₂ | Ekelhaft faul | --- | --- | 88,15 |
| Pyridin | C₆H₅N | Unangenehm reizend | 0,0037 | --- | 79,1 |
| Skatole | C₉H₉N | Ekelhaft fäkal | 0,0012 | 0,47 | 131,2 |

## Patentansprüche

1. Luftschadstoffneutralisationsreiniger (Air Cleaner) zur Absorption von Schadstoffen und Neutralisierung von Geruchsstoffen zur Verbesserung der Raumluft,
**gekennzeichnet durch**
- einen Aminosäurenkomplex zumindest umfassend Bio-L-Arginin, L-Lysin, L-Glutamin und L-Prolin und
- ein Compound auf Zinkrizinoleat-Basis von bis zu 10 %,
- wobei die Gesamtrezeptur ausschließlich natürliche Inhaltsstoffe aufweist und
- die Rezeptur zumindest umfasst:
- 70,00% bis 90,00% Wasser,
- 6,00% bis 15,00% Bio-Ethanol,
- 0,50% bis 3,00% Bio-L-Arginin,
- 0,05% bis 1,50% L-Glutamin,
- 0,30% bis 2,00% L-Lysin,
- 0,04% bis 1,00% L-Prolin,
- 2,00% bis 5,00% polyglycerol sowie
- 0,20% bis 1,00% Zitronensäure und
- 5,00% bis 10,00 % Zinkrizinoleat Coumpond.

2. Luftschadstoffneutralisationsreiniger (Air Cleaner) nach Anspruch 1,
**dadurch gekennzeichnet, dass**
das enthaltene polyglycerol als Emulgator und/oder zur Regelung der Viskosität anteilig in der Rezeptur vorhanden ist.

3. Luftschadstoffneutralisationsreiniger (Air Cleaner) nach Anspruch 1 oder 2,
**dadurch gekennzeichnet, dass**
die enthaltene Zitronensäure als pH-Wert-regulierender Inhaltsstoff in der Rezeptur vorhanden ist.

4. Luftschadstoffneutralisationsreiniger (Air Cleaner) nach einem der vorangegangenen Ansprüche,
**dadurch gekennzeichnet, dass**
ein Chelatbildner im Zinkrizinoleat Compound enthalten ist, der dessen Geruchsneutralisationseigenschaften aktiviert und die Grenzflächenaktivität erhöht.

5. Luftschadstoffneutralisationsreiniger (Air Cleaner) nach Anspruch 4,
**dadurch gekennzeichnet, dass**
der Chelatbildner einen Mengenanteil Tetrasodium Glutamate Diacetate von etwa 0,8% bis 2,0% an der Gesamtmenge der Mischung aufweist.

6. Luftschadstoffneutralisationsreiniger (Air Cleaner) nach einem der vorangegangenen Ansprüche,
**dadurch gekennzeichnet, dass**
als Duftstoff zumindest ein natürliches und/oder ätherisches Öl anteilig in der Rezeptur vorhanden ist.

7. Luftschadstoffneutralisationsreiniger (Air Cleaner) nach einem der vorangegangenen Ansprüche,
**dadurch gekennzeichnet, dass**
in der Rezeptur 99,5 % nach ECOCERT Standard "Wasch und Reinigungsmittel" zertifizierte Inhaltsstoffe enthalten sind.

## Claims

1. Air pollutant neutralizing cleaner (air cleaner) for absorption of pollutants and neutralization of odorous substances for the improvement of indoor air,
**characterized by**
- an amino acid complex at least comprising bio-L-arginine, L-lysine, L-glutamine and L-proline and
- a compound based on zinc ricinoleate of up to 10%,
- wherein the overall formulation comprises exclusively natural ingredients and
- the formulation contains at least:
- 70.00% to 90.00% of water,
- 6.00% to 15.00% of bioethanol,
- 0.50% to 3.00% of bio-L-arginine,
- 0.05% to 1.50% of L-glutamine,
- 0.30% to 2.00% of L-lysine,
- 0.04% to 1.00% of L-proline,
- 2.00% to 5.00% of polyglycerol and also
- 0.20% to 1.00% of citric acid and
- 5.00% to 10.00% of zinc ricinoleate compound.

2. Air pollutant neutralizing cleaner (air cleaner) according to Claim 1,
**characterized in that**
the proportion of polyglycerol contained in the formulation is present as an emulsifier and/or for regulation of viscosity.

3. Air pollutant neutralizing cleaner (air cleaner) according to Claim 1 or 2,
**characterized in that**
the citric acid contained in the formulation is present as a pH-regulating ingredient.

4. Air pollutant neutralizing cleaner (air cleaner) according to any of the preceding claims,
**characterized in that**
a chelating agent is contained in the zinc ricinoleate compound, which activates the odour neutralizing properties of the latter and enhances the surface activity.

5. Air pollutant neutralizing cleaner (air cleaner) according to Claim 4,
**characterized in that**
the chelating agent has a proportion of tetrasodium glutamate diacetate of about 0.8% to 2.0% in the total amount of the mixture.

6. Air pollutant neutralizing cleaner (air cleaner) according to any of the preceding claims,
**characterized in that**
a proportion of at least one natural and/or essential oil is present in the formulation as a fragrance.

7. Air pollutant neutralizing cleaner (air cleaner) according to any of the preceding claims,
**characterized in that**
the formulation contains 99.5% of ingredients certified in accordance with the Ecocert standard "Wash and cleaning agents".

## Revendications

1. Purificateur d'air à neutralisation de polluants (Air Cleaner) pour l'absorption de polluants et la neutralisation de substances odorantes en vue de l'amélioration de l'air ambiant,
**caractérisé par**
- un complexe d'acides aminés comprenant au moins de la bio-L-arginine, de la L-lysine, de la L-glutamine et de la L-proline et
- un composé à base de ricinoléate de zinc à raison de jusqu'à 10 %,
- la formulation totale présentant exclusivement des ingrédients naturels et
- la formulation comprenant au moins :
- 70,00 % à 90,00 % d'eau,
- 6,00 % à 15,00 % de bio-éthanol,
- 0,50 % à 3,00 % de bio-L-arginine,
- 0,05 % à 1,50 % de L-glutamine,
- 0,30 % à 2,00 % de L-lysine,
- 0,04 % à 1,00 % de L-proline,
- 2,00 % à 5,00 % de polyglycérol ainsi que
- 0,20 % à 1,00 % d'acide citrique et
- 5,00 % à 10,00 % de composé à base de ricinoléate de zinc.

2. Purificateur d'air à neutralisation de polluants (Air Cleaner) selon la revendication 1,
**caractérisé en ce que**
le polyglycérol contenu est présent de manière proportionnée dans la formulation en tant qu'émulsifiant et/ou pour la régulation de la viscosité.

3. Purificateur d'air à neutralisation de polluants (Air Cleaner) selon la revendication 1 ou 2,
**caractérisé en ce que**
l'acide citrique contenu est présent dans la formulation en tant qu'ingrédient de régulation du pH.

4. Purificateur d'air à neutralisation de polluants (Air Cleaner) selon l'une des revendications précédentes, **caractérisé en ce que**
un agent chélatant est contenu dans le composé à base de ricinoléate de zinc, qui active ses propriétés de neutralisation des odeurs et qui augmente l'activité tensioactive.

5. Purificateur d'air à neutralisation de polluants (Air Cleaner) selon la revendication 4,
**caractérisé en ce que**
l'agent chélatant présente une proportion de diacétate de glutamate tétrasodique d'environ 0,8 % à 2,0 % de la quantité totale du mélange.

6. Purificateur d'air à neutralisation de polluants (Air Cleaner) selon l'une des revendications précédentes, **caractérisé en ce que**
une huile naturelle et/ou essentielle est présente de manière proportionnée dans la formulation en tant que parfum.

7. Purificateur d'air à neutralisation de polluants (Air Cleaner) selon l'une des revendications précédentes, **caractérisé en ce que**
la formulation comprend 99,5 % d'ingrédients certifiés selon la norme ECOCERT "Produits de nettoyage".
